Europäisches Patentamt

European Patent Office

Office européen des brevets

(11) Publication number: **0 430 398 A2**

## EUROPEAN PATENT APPLICATION

(21) Application number: 90308050.5

(22) Date of filing: 23.07.90

(51) Int. Cl.5: **A61M 5/14, A61M 39/00**

(30) Priority: 28.11.89 US 441954

(43) Date of publication of application:
05.06.91 Bulletin 91/23

(84) Designated Contracting States:
DE FR GB Bulletin 2

(71) Applicant: IMED CORPORATION
9925 Carroll Canyon Road
San Diego California 92131(US)

(72) Inventor: Richmond, Frank M.
205 A. Grant Street
Harvard, Illinois 60033(US)
Inventor: Vanderveen, Timothy
17197 Tam O' Shanter Drive
Poway, California 92067(US)
Inventor: Kimes, Richard
2935 Via Emerado
Carlsbad, California 92009(US)

(74) Representative: Dealtry, Brian et al
Eric Potter & Clarkson St. Mary's Court St.
Mary's Gate
Nottingham NG1 1LE(GB)

(54) Multiline check valve assembly.

(57) A multiline check valve assembly has an elongated body which defines a fluid passageway and which is formed with a plurality of valve cavities that are in fluid communication with the fluid passageway. Each valve cavity has a resilient valve element disposed within the cavity and each valve element rests in the cavity with one end against a protrusion disposed in the cavity for centering the element within the cavity. The resilient valve element is biased to urge its other end against a valve seat that surrounds an opening to the valve cavity to create a fluid tight seal with the valve seat and close the opening. In addition, and access port is formed circumferentially around both the valve seat and opening to establish a fluid communication path through the access port to the fluid passageway in the body when the valve element is separated from the valve seat. Specifically, when an appropriate fluid line connector is inserted into the access port to urge against the resilient valve element, the valve element is deformed away from the valve seat to complete the fluid communication path between the access port and the fluid passageway.

Fig 2

The present invention relates generally to assemblies for permitting the infusion of a fluid into an IV line and more particularly to a check valve assembly. More specifically, the present invention relates to check valve assemblies which may be interconnected between a primary fluid passageway and a plurality of secondary fluid sources. The present invention is particularly, though not exclusively, useful for infusing fluids into a patient from several sources through a single IV administration line.

The use of volumetric pumps to assist in the infusion of medications to patients is well established in the medical field. Several devices have been proposed for this purpose. For example, US Patent No 3,985,133, claims and discloses a volumetric pump that accurately infuses medications to a patient. As another example, US Patent No 4,617,014 discloses a linear peristaltic pump which also accurately infuses medications to a patient. Such systems, however, are typically designed for the infusion of medications through a single fluid line. Thus, whenever an additional medical fluid needs to be infused, either the pump must be temporarily shut down while the fluid container in the existing IV system is changed or a separate IV system must be set up. In either case, there may be unacceptable delays. Moreover, additional IV lines may need to be inserted into the patient, causing patient discomfort and compounding the problems associated with IV line management and maintenance. Such problems, however, can be avoided if the new medical fluid can be introduced into the existing fluid delivery system without disassembling the existing system.

Several valving devices have been proposed in the prior art for diverting fluid flow from one path to another. One such valve is the manually-operable three-way valve device disclosed in US Patent No 3,057,370. Other valves, such as the check valve disclosed in US Patent No 3,352,531 have been proposed which are opened by cooperation with an external structure, such as the tip of a syringe, to establish a fluid passageway. additional examples of such devices in the medical field are US Patent No 3,385,301 and US Patent No 3,799,171. Another depressor activated device is the valve disclosed in US Patent No 3,965,910 which defines a separate passageway for the addition of a second fluid into an existing fluid pathway during engagement of the depressor. Further, these are numerous examples of so called piggy back systems which allow for the continuity of infusion from two separate fluid sources. The device disclosed in US Patent No 4,533,347 and assigned to the same assignee as the present invention is an example of one such system. There is, however, still the need for continuity of infusion from more than one secondary source. Also, there is a need for an automatic return to the normal fluid flow of the pre-existing fluid pathway when flow from several fluid sources has been completed.

It is an object of the present invention to provide an improved assembly for permitting infusion of fluid into an IV line.

According to one aspect of this invention there is provided an assembly for permitting the infusion of fluid into an IV line, said assembly comprising; a valve body defining a fluid passageway for permitting fluid communication with said IV line, and defining a fluid pathway in fluid communication with said fluid passageway; deformable means positioned against said body and being deformable between a first configuration to occlude said fluid pathway and a second configuration to open said fluid pathway; and holding means for holding said deformable means against said body.

According to another aspect of this invention there is provided a check valve assembly connecting the line of a secondary fluid source in fluid communication with a primary IV infusion line which comprises:

a valve body defining a fluid passageway, said body having a valve cavity in fluid communication with said passageway, said valve cavity having a bottom and an opening spaced from said bottom, said opening having a valve seat circumscribing said opening;

a resilient valve element disposed in said valve cavity between said bottom and said opening, said valve element being deformable between a first configuration wherein said valve element is in fluid sealable contact with said valve beat to occlude said opening and a second configuration wherein said valve element is distanced from said seat to establish a fluid pathway through said opening into said passageway; and

means projecting from said bottom of said valve cavity and engageable with said valve element for substantially centering said valve element within said valve chamber.

Preferably said body comprises a plurality of said valve cavities and associated said valve elements.

Preferably said valve element has a skirt defining a recess, and said projecting means may comprise a protrusion for engaging said recess of said valve element to center and orient said element in said cavity.

Said valve body may comprise a top plate and a base portion, said opening being formed on said top plate, said top plate may have an access port circumferentially surrounding said valve seat and said opening for receiving said line from said secondary source.

Said valve element may be formed with a shoulder for sealable contact with said valve seat. Said valve element may be further formed with an extension insertable into said access port which extends from said shoulder opposite said skirt.

Said valve element may be biased into said first configuration and deformable into said second configuration.

Said valve element may be deformable into said second configuration by fluid pressure.

Said valve element may be deformable into said second configuration by said line from said secondary source.

According to another aspect of the invention there is provided an assembly for permitting the infusion of fluid into an IV line which comprises:

a base which defines a fluid passageway for permitting fluid communication with said IV line;

an access port mounted on said base, said access port defining a fluid pathway in fluid communication with said fluid passageway;

deformable means having a first end positioned against said base and having a second end positioned against said access port to block said fluid pathway when said deformable means is in a first configuration and to open said fluid pathway when said deformable means is in a second configuration; and

means on said base to hold said first end of said deformable means stationary relative to said base.

Said base may further comprise a valve cavity in fluid communication with said passageway. Said valve cavity may be formed with an opening surrounded by a valve seat, said opening being in fluid communication with said access port and said valve cavity also being formed with means for centering said deformable means within said valve cavity.

Said deformable means may comprise a resilient valve element disposed in said valve cavity, said valve element being deformable between said first and second configurations.

Said centering means may comprise a skirt formed on said valve element has a skirt defining a recess, said centering means may further comprise a protrusion formed on said valve cavity opposite said valve seat for engaging said recess of said valve element to center and orient said element in said cavity.

Said base may comprise a plurality of said valve cavities and associated said valve elements.

Said valve element may be formed with a shoulder for forming a fluid sealable contact with said valve seat said valve element may be further formed with an extension insertable into said access port which extends from said shoulder opposite said skirt.

Said valve element may be biased into said first configuration, and deformable into said second configuration.

Said valve element may be deformable into said second configuration by fluid pressure.

Said valve element may be deformable into said second configuration by inserting a fitting into said opening to urge against said valve element.

According to another aspect of this invention there is provided a method for infusing fluid from a secondary source into an IV line which is carrying fluid from a primary source, which comprises the steps of:

(a) engaging said primary source in fluid communication with a device comprising:

a base valve body defining a fluid passageway for permitting fluid communication from said primary source through said IV line, said body having a valve cavity in fluid communication with said passageway, said valve cavity having a bottom and an opening spaced from said bottom, said opening having a valve seat circumscribing said opening; a resilient valve element disposed in said cavity, said valve element being deformable between a first configuration wherein said valve element is in fluid sealable contact with said valve seat and a second configuration wherein said valve element is distanced from said seat to establish a fluid pathway through said opening into said passageway; and

(b) inserting an adaptor connected in fluid communication with said secondary source into said opening to deform said valve element into its second configuration to thereby establish a fluid pathway between said secondary source and said fluid passageway.

Accordingly, it is an advantage of the preferred embodiment of the present invention that it provides a multiline check valve which is simple in operation and allows for easy engagement of a plurality of secondary fluid sources into a pre-existing fluid flow line. Another advantage of the present invention is that it provides a cost effective disposable valve for use with a pumping system that will permit the accurate delivery of fluid from a plurality of separate fluid sources. It is still another advantage of the present invention that it provides a valve which can reestablish the pre-existing fluid pathway after the introduction of fluid from the second sources has been completed.

The preferred embodiment of the novel multiline check valve assembly in accordance with the present invention comprises an elongated body which defines a longitudinal fluid passageway. Additionally, the body may have a plurality of serially aligned cylindrical-shaped valve cavities which are formed in the body. These cavities may be formed generally transverse to the fluid passageway and in fluid communication with the fluid passageway. Specifically, each valve cavity may have an inlet from the passageway and an outlet to the passageway which together allows for fluid flow through the valve cavity. Within each valve cavity may be four ribs, which may be formed on the walls of the cavity in an orientation that is substantially perpendicular to the direction of fluid flow through the passageway. More particularly, one pair of ribs may be formed immediately adjacent to the passageway inlet to the cavity with the ribs preferably disposed on opposite sides of the inlet from each other. Similarly, the other pair of ribs may be formed on the cavity's inside wall immediately adjacent to the passageway outlet. Again the ribs are preferably disposed on opposite sides of the outlet from each other. The bottom wall of the cavity may be formed with a raised protrusion and the top of the valve cavity may be formed with a valve seat which surrounds and defines an opening.

Disposed within each valve cavity may be a resilient valve element which may have one end that straddles the protrusion of the valve cavity and may have its other end positioned to urge against the valve seat. More particularly, each valve element may be formed with a skirt section that defines a recess. This recessed portion of the valve element skirt may be insertable over the protrusion of the valve cavity to center the valve element within the valve cavity. In order to create a fluid seal, the valve element may be formed with a shoulder which normally rests against the valve seat. Further, the valve element may have a portion which extends through the opening circumscribed by the valve seat. In the preferred embodiment, this extended portion projects into an access port which is formed on the valve body around the valve seat. The access port may thereby provide for an engagement between the valve body and an appropriate fluid line connector to establish fluid communication between a secondary fluid source and the fluid passageway in the valve body.

For the operation of the check valve assembly of the preferred embodiment, it is to be appreciated that the resilient valve element is normally biased into a first, or seated, configuration wherein a fluid seal is established between the shoulder of the valve element and the valve seat. When a fluid line connector is inserted into the access Port, however, the connector may urge against the extended portion of the valve element to deform the valve element into its second, or unseated, configuration. In this second configuration, the shoulder of the valve element may be separated from the valve seat to establish a fluid communication pathway between the fluid passageway in the valve body and a secondary fluid source which may be in fluid communication with the connector. When the valve element is so deformed, it may happen that the ribs of the valve cavity contact the deformed valve element to prevent occlusion of the fluid passageway inlet or outlet by the deformed valve element.

For the purposes of the present invention, the valve body may either be of unitary construction or, preferably, may be formed by the attachment of a top plate onto a base portion. With the embodiment wherein a top plate is joined to a base, the valve cavities and the interconnecting fluid passageway are formed on the base. Also, with this embodiment, the access ports are formed on the top plate. When the top plate and base are joined, there may be a mating engagement of the access ports with the valve cavities.

Reference is now made to the accompanying drawings in which:

Figure 1 is a front elevational view of the novel multiline valve assembly in its intended environment;

Figure 2 is a perspective view of the novel multiline valve assembly;

Figure 3 is an exploded side view of the novel multiline valve assembly;

Figure 4 is a top cross-sectional view of the body portion of the novel multiline valve assembly as seen along the line 4-4 in Figure 2;

Figure 5 is a cross-sectional view of one valve cavity as seen along the line 5-5 in Figure 2, with the valve element in its seated configuration; and

Figure 6 is a cross-sectional view of the valve cavity as seen in Figure 5, with the valve element in its unseated configuration.

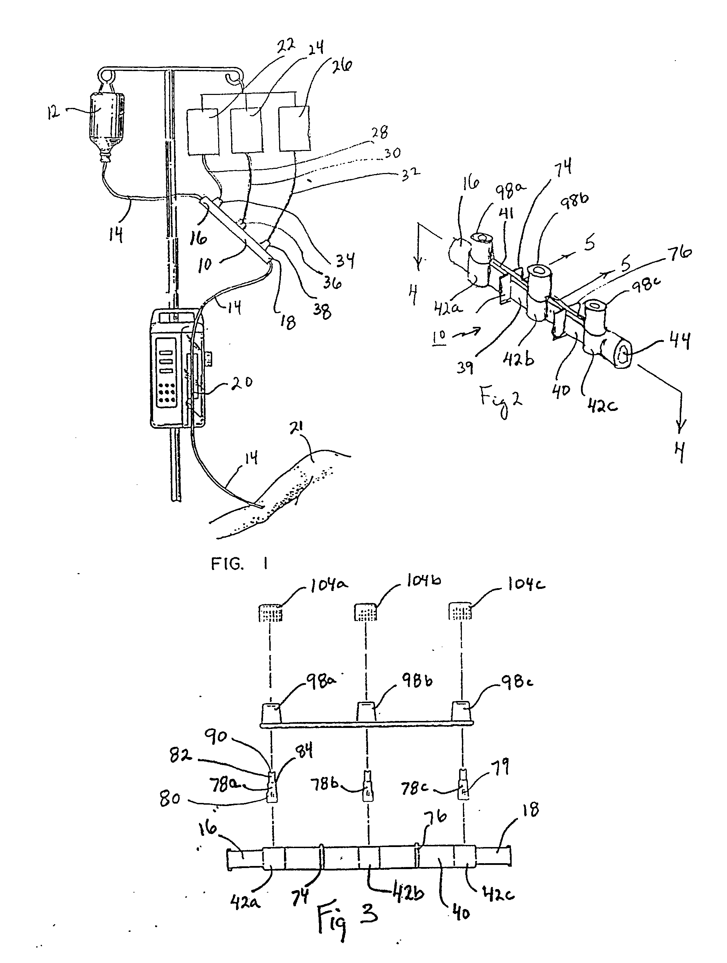

Referring initially to Figure 1, a multiline valve assembly 10 is shown operatively connected in fluid communication between a primary fluid source 12 and an IV infusion pump 20 through a line 14. More particularly, fittings 16 and 18 permanently or detachably connect the assembly 10 with the line 14. For purposes of the present invention, any suitable IV infusion pump 20 may be operatively connected with line 14 downstream of multiline valve assembly 10 for infusing fluid which passes through multiline valve assembly 10 into a patient 21. Also shown connected to assembly 10 are a plurality of secondary fluid sources 22, 24, add 26, which are respectively attached to assembly 10 through lines 28, 30 and 32 by fittings 34, 36, and 38, respectively. It will be understood by the skilled artisan that the fittings 16, 18, 28, 30, and 32 are preferably standard Lure IV fittings and that fittings 16 and 18 may interchangeably be either male or female fittings.

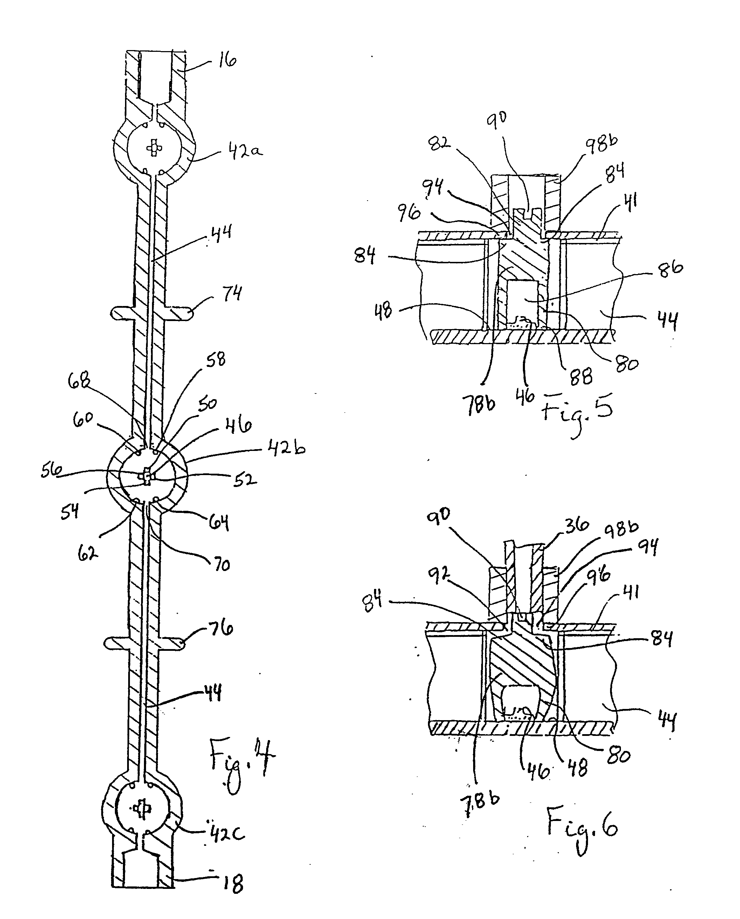

The overall structure of valve assembly 10 can purhaps be best appreciated with reference to both Figure 2 and Figure 3. In Figure 2 it is seen that valve assembly 10 has an elongated valve body 39 which is established by joining a base 40 with a top plate 41 by any means well known in the art, such as by solvent bonding, sonic sealing, or RF sealing.

As shown, a series of valve cavities 42a, b and c are formed along the valve body 39 and a fluid passageway 44 is established longitudinally within the valve body 39. To allow fluid flow through valve assembly 10, fluid passageway 44 is in fluid communication with each of the valve cavities 42a, b, and c.

In figure 4 it is seen that each cavity 42 of valve assembly 10 is formed with a centering protrusion 46. As seen by cross referencing Figure 4 with Figure 5 or Figure 6, each protrusion 46, is elevated with respect to the bottom 48 of the cavity 42, and includes radial ledges 50, 52, 54, and 56 which extend from the centering protrusion 46. Like protrusion 46 the radial ledges 50, 52, 54 and 56 are elevated relative to bottom 48 of valve cavity 42. Using cavity 42b as an example Figure 4 also shows four longitudinal ribs 58, 60, 62, and 64, which are formed on the walls 66 of valve cavity 42b. More particularly, the ribs 58, 60, are formed immediately adjacent to the cavity inlet 68 while ribs 62, 64 are formed immediately adjacent to the cavity outlet 70 of fluid pasageway 44. All ribs 58, 60, 62 and 64 protrude from walls 66 into valve cavity 42b. It will be further appreciated in cross-reference to Figures 5 and 6 that ribs 58, 60, 62, 64, as well as cavity inlets 68, 70, extend from the bottom 48 of valve cavity 42 to the top 72 of valve cavity 42.

Referring back to Figure 2, it may be seen that base 40 may also be formed with stand off adaptors 74 and 76 for facilitating engagement between multiline valve assembly 10 and other appropriate IV infusion system components as required. Finally, it will be appreciated that the base 40, as well as the other components of multiline valve assembly 10, may be composed of any suitable material, such as lightweight plastic. Importantly, the selected material should both be structurally strong, and compatible with the fluid medicament being infused.

The interaction of components of valve assembly 10 is best indicated in Figure 3, which shows a plurality of resilient valve elements 78a, b and c that are respectively positioned in each of the valve cavities 42a, b and c. Each of the valve elements 78a, b and c, as seen in Figures 3 and 5, is formed with a hollow skirt portion 80 and an extension 82. Further, each valve element 78a, b and c is formed with a seating shoulder 84 located between skirt portion 80 and extension 82. Additionally, with specific regard to valve cavity 42b, skirt portion 80 forms a recess 86 (shown in cross-section in Figure 5) which establishes an interference fit between valve element 78b and protrusion 46 in valve cavity 42b. Specifically, the recess 86 is positioned in a surrounding relationship with the centering protrusion 46 and is positioned for an interference fit with the radial ledges 50, 52, 54, 56. When so positioned, it will be understood that the bottom edge 88 of valve element 78 rests against the bottom 48 of valve cavity 42. As will readily be appreciated, this disposition of valve element 78a, b and c around their respective centering protrusions 46 substantially centers each valve element 78a, b and c within a respective valve cavity 42a, b and c.

It is also seen in reference to Figure 2 that the extension 82 of valve element 42 may be formed with a groove 90. In addition, each valve element 78a, b and c may be formed with a plurality of ridges 79 which extend longitudinally along the skirt 80 of the valve element 78a, b and c. Finally, the material of valve element 78a, b and c is selected to be resilient and deformable, so that it regains its non-deformed shape shown in Figures 3 and 5 upon removal of any deforming force. For the embodiment shown in Figure 2, valve element 42 is composed of an elastomeric material well known in the art, such as rubber.

Figures 2 and 3 also show that while valve body 39 may be of unitary construction, preferably, a top plate 41 and base 40 are formed as a separate components of multiline valve assembly 10. When formed as a separate component of assembly 10, top plate 41 may be attached to base 40 by any suitable means, such as by gluing, solvent bonding or sonic welding, to cover the passageway 44 formed through valve body 39. In either case, top plate 41 is formed with a plurality of openings 94, which are each circumscribed by a valve seat 96 for forming a fluid tight seal with the respective shoulders 84 of associated valve elements 78. More specifically, each valve element 78 is biased to its seated or non-deformed configuration, as shown in Figure 5, wherein shoulder 84 is disposed in fluid sealable contact around the circumference of its associated valve seat 96 to prevent fluid communication through the respective opening 94. While shoulder 84 and seat 96 are shown in Figure 5 to be substantially parallel to the dimension of elongation of assembly 10, it will be understood that the shoulder 84 (and, correspondingly, seat 96) may be tapered relative to the dimension of elongation of assembly 10. It may now be appreciated that by forming valve element 78 of a resilient material, valve element 78 may be deformed from its seated configuration described above to an unseated or deformed configuration wherein shoulder 84 is distanced from its associated valve seat 96. Such a deformed configuration is shown in Figure 6. As shown, when valve element 78 a, b or c is deformed, a pathway 92 is established through opening 94 which permits fluid communication through top plate 41 into fluid passageway 44.

Figure 2 also shows access ports 98a, b and c extending respectively from each opening 94 to receive the appropriate fitting 34, 36, or 38 shown in Figure 1. With fitting 34, 36 or 38 properly engaged with access ports 98a, b or c, a fluid communication pathway 92 is established between the associated secondary fluid source 22, 24, or 26 and the fluid passageway 44. This connection is best seen in Figure 6. Finally, Figure 3 shows a plurality of removable covers 104a, b and c that protect access port 98a, b and c when multiline valve apparatus 10 is not in use. It will therefore be appreciated that the covers 104 keep dirt and other material from entering multiline valve assembly 10 when assembly 10 is not in use.

## OPERATION

In the operation of the novel multiline valve assembly 10, reference is initially made to Figures 1 and 2 where it is seen that multiline valve assembly 10 is attached to line 14 through fittings 16, 18, respectively. In this configuration, a single path for fluid communication between primary source 12 and pump 20 exists through passageway 44. Furthermore, in reference to Figure 5, it may be seen that when no secondary fluid source is attached to an access port 98a, b or c, the respective valve element 78a, b or c is biased into its undeformed, or stated configuration. As seen in Figure 5, when valve element 78 is in this seated configuration, its shoulder 84 forms a fluid tight seal around the valve seat 96 of top plate 41, thereby preventing fluid communication into fluid passageway 44 through opening 94. When cover 104 is removed, however, and a secondary fluid source (e.g., source 24) is attached to multiline valve assembly 10 as shown in Figure 6, the respective secondary fitting (e.g., fitting 36) extends into access port 98b and urges against the extension 82 of valve element 78b. By so urging, the fitting 36 deforms valve element 78b into its unseated or deformed configuration, wherein a fluid pathway 92 is formed through opening 94 to connect secondary source 22 in fluid communication with fluid passageway 44. Alternativly, it is to be understood that resilient valve element 78b may be constructed to deform, if desired, when fluid pressure from source 22 acts against extension 82 and shoulder 84 of resilient valve element 78b. In either case, it may now be appreciated that when valve element 78b is so deformed, one or more of the ribs 58, 60, 62, 64 may contact skirt portion 80 of valve element 78 to preclude occlusion of inlet 68 or outlet 70 by valve element 78. Thus, fluid passageway 44 remains unoccluded to permit fluid communication between primary source 12 and pump 20 when valve element 78b is in its deformed configuration to simultaneously connect fluid source 24 in fluid communication with pump 20.

It is to be appreciated that, although the disclosure here has focused primarily on the structure and interaction of valve cavity 42b and valve element 78b, the structure and interaction of valve cavities 42a and c with valve elements 78a and c are in all essential aspects equivalent.

While the particular multiline valve assembly as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as defined in the appended claims.

## Claims

1. An assembly for permitting the infusion of fluid into an IV line, said assembly comprising; a valve body defining a fluid passageway for permitting fluid communication with said IV line, and defining a fluid pathway in fluid communication with said fluid passageway; deformable means positioned against said body and being deformable between a first configuration to occlude said fluid pathway and a second configuration to open said fluid pathway; and holding means for holding said deformable means against said body.

2. An assembly according to claim 1 wherein said body has a valve cavity in fluid communication with said fluid pathway via an opening surrounded by a valve seat, and being in fluid communication with said passageway.

3. An assembly according to claim 2 wherein said valve body comprises a top plate and a base portion, said opening being formed in said top plate, and said top plate having an access port defining said fluid pathway, said access port surrounding said valve seat and said opening.

4. An assembly according to claim 3 wherein said deformable means comprises a resilient valve element disposed in said valve cavity, said valve element being deformable between said first and second configurations.

5. An assembly according to claim 4 holding means comprises a skirt formed on said valve element, said skirt defining a recess, and said holding means further comprising centering means projecting from said valve cavity for engaging said recess of said valve element to center and orient said element in said cavity.

6. An assembly according to claim 5 wherein said valve element is formed with a shoulder for sealable contact with said valve seat, said valve element being further formed with an extension insertable into said access port which extends from said shoulder opposite said skirt.

7. An assembly according to claim 4, 5 or 6 wherein said valve element is biased into said first configuration and deformable into said second configuration.

8. An assembly according to claim 7 wherein said valve element is deformable into said second configuration by fluid pressure.

9. An assembly according to claim 7 wherein said valve element is deformable into said second configuration by inserting a fitting into said opening to urge against said valve element.

10. An assembly according to any of claims 4 to 9 wherein said base portion comprises a plurality of said valve cavities and associates said valve elements.

11. A method for infusing fluid for a secondary source into an IV line which is carrying fluid from a primary source, which comprises the steps of:

(a) engaging said primary source in fluid communication with a device comprising:

a base valve body defining a fluid passageway for permitting fluid communication from said primary source through said IV line, said body having a valve cavity in fluid communication with said passageway, said valve cavity having a bottom and an opening spaced from said bottom, said opening having a valve seat circumscribing said opening; a resilient valve element disposed in said cavity, said valve element being deformable between a first configuration wherein said valve element is in fluid sealable contact with said valve seat and a second configuration wherein said valve element is distanced from said seat to establish a fluid pathway through said opening into said passageway; and

(b) inserting an adaptor connected in fluid communication with said secondary source into said opening to deform said valve element into its second configuration to thereby establish a fluid pathway between said secondary source and said fluid passageway.

FIG. 1

Fig 2

Fig 3

Fig. 4

Fig. 5

Fig. 6